(19) Europäisches Patentamt
European Patent Office
Office européen des brevets

(11) Publication number: **0 024 873**
**A1**

# (12) EUROPEAN PATENT APPLICATION

(21) Application number: 80302864.6

(22) Date of filing: 19.08.80

(51) Int. Cl.[3]: **C 07 D 401/12**
C 07 D 401/14, C 07 D 403/12
C 07 D 403/14, C 07 D 405/12
C 07 D 405/14

(30) Priority: 21.08.79 GB 7929012

(43) Date of publication of application:
11.03.81 Bulletin 81/10

(84) Designated Contracting States:
AT BE CH DE FR GB IT LI LU NL SE

(71) Applicant: SMITH KLINE & FRENCH LABORATORIES LIMITED
Mundells
Welwyn Garden City Hertfordshire, AL7 1EY(GB)

(72) Inventor: Brown, Thomas Henry
17 Godfries Close
Tewin Hertfordshire(GB)

(72) Inventor: King, Ronald Joseph
3 Red Lion Yard
Wareside Hertfordshire(GB)

(72) Inventor: White, George Raymond
16 Ashley Gardens
Harpenden Hertfordshire(GB)

(74) Representative: Waters, David Martin, Dr. et al,
P.O. Box 39
Welwyn Garden City, Hertfordshire AL7 1EY(GB)

## (54) A process for preparing pyrimidones.

(57) A process is disclosed for preparing compounds of formula (1) :-

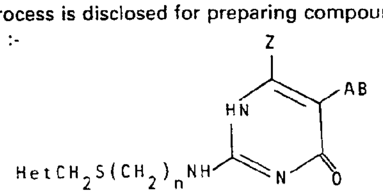

(1)

and acid addition salts thereof where Het is an optionally substituted 5- or 6- membered fully unsaturated nitrogen containing heterocyclic group where the substituents (which can be the same or different) are one or more $C_{1-4}$ alkyl, trifluoromethyl, halogen, hydroxy, $C_{1-4}$ alkoxy or amino groups; n is 2 or 3; Z is hydrogen or $C_{1-4}$ alkyl; A is $C_{1-5}$ alkylene optionally interrupted with oxygen or sulphur and B is hydrogen, methyl, $C_{3-6}$ cycloalkyl, an optionally substituted heteroaryl group where the substituents (which can be the same or different) are one or more $C_{1-4}$ alkyl, $C_{1-4}$ alkoxy or hydroxy groups; or B is a naphthyl, 5- or 6-(2,3-dihydro-1,4-benzodioxinyl) or a 4- or 5-(1,3-benzodioxolyl) group or an optionally substituted phenyl group where the substituents (which can be the same or different) are one or more $C_{1-4}$ alkyl, $C_{1-4}$ alkoxy, halogen, aryl-($C_{1-4}$ alkoxy), hydroxy, $C_{1-4}$ alkoxy-$C_{1-4}$ alkoxy, trifluoromethyl, di($C_{1-4}$ alkyl) amino, phenoxy, halophenyl, or $C_{1-4}$ alkoxyphenyl groups which comprises reacting a compound of formula (4) :-

$$Het-CH_2L$$

(4)

or an acid addition salt thereof where Het is as defined with reference to formula (1) provided that any substituents which interfere with the reaction are protected and L is a group displacable with thiol with a compound of formula (3) :-

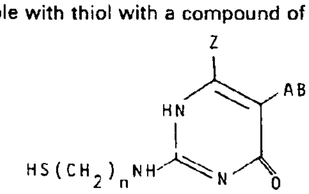

(5)

or an acid addition salt thereof where n, Z, A and B are as defined with reference to formula (1) provided that any phenolic hydroxy groups within B are optionally protected, and thereafter removing any protecting groups.

EP 0 024 873 A1

# A PROCESS FOR PREPARING PYRIMIDONES

This invention relates to a process for preparing certain pyrimidone derivates.

European Patent Application No 0004793 describes a process for preparing *inter alia* pyrimidone derivatives of general formula (1) :-

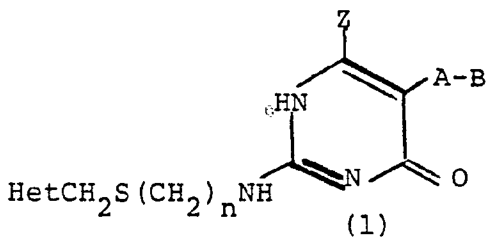

(1)

where Het is an optionally substituted 5- or 6- membered fully unsaturated nitrogen containing heterocyclic group in which the substituents (which can be same or different) are one or more $C_{1-4}$ alkyl, trifluoromethyl, halogen, hydroxy, $C_{1-4}$ alkoxy, or amino groups; n is 2 or 3; Z is hydrogen, or $C_{1-4}$ alkyl; A is $C_{1-5}$ alkylene optionally interrupted by oxygen or sulphur and B is hydrogen, methyl, $C_{3-6}$ cycloalkyl, an optionally substituted heteroaryl group where the substituents (which can be the same or different) are one or more $C_{1-4}$ alkoxy or hydroxy groups; or B is a naphthyl, 5- or 6-(2,3-dihydro-1,4-benzodioxinyl) or a 4- or 5- (1,3-benzodioxolyl) group or an optionally substituted phenyl group where the substituents (which are the same of different) are one or more $C_{1-4}$ alkyl, $C_{1-4}$ alkoxy, halogen, aryl($C_{1-4}$alkoxy), hydroxy $C_{1-4}$alkoxy-$C_{1-4}$alkoxy, trifluoromethyl, di($C_{1-4}$alkyl) amino, phenoxy, halophenyl or $C_{1-4}$alkoxyphenyl groups; where an amine of formula (2), in which Het, and n are as defined for formula (1), is reacted with a 2-nitroamino-pyrimidone of formula (3), in which Z, A and B are

$$Het - CH_2\ S(CH_2)_n\ NH_2$$

(2)

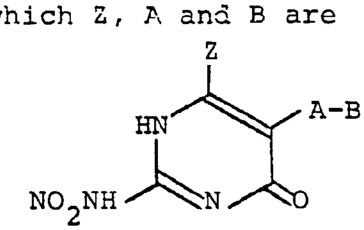

(3)

as defined for formula (1).

Many of the compounds of formula (1) are described in German Offenlegungsschriften 2643670 and 2658267 as having histamine $H_1$- and $H_2$- antagonist activity and in German Offenlegungsschrift 2421548 as having histamine $H_2$- antagonist activity. Histamine $H_2$- antagonists and compounds which have histamine $H_1$- and $H_2$- antagonist activity are useful as inhibitors of gastric acid secretion, as antiinflammatory agents and as agents which inhibit the effects of histamine on blood pressure.

According to the present invention, the compounds of formula (1) above and acid additions salts thereof, are prepared by a process which comprises reacting a compound of formula (4) :-

$$Het-CH_2L$$

(4)

or an acid addition salts thereof where Het is as defined with reference to formula (1) provided that any substituents which interfere with the reaction are protected, with a compound of formula (5) :-

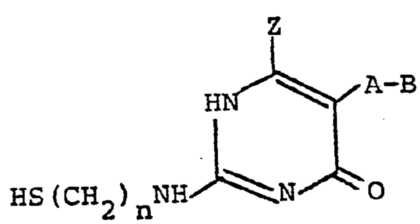

(5)

or an acid addition salt thereof where n, Z, A and B are as defined with reference to formula (1) provided that any phenolic hydroxy groups within B are optionally protected; and thereafter removing any protecting groups, and optionally converting the product into an acid addition sa

The compounds of formulae (1) and (5) are shown and described as 4-pyrimidone derivatives but is is appreciated tha they can exist in equilibrium with other tautomeric forms as follows :-

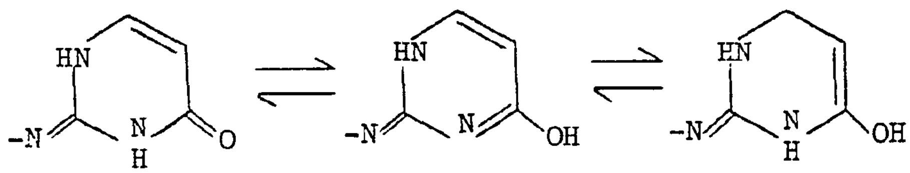

Examples of the optionally substituted heterocyclic groups which Het represents are optionally substituted imidazolyl, pyridyl, thiazolyl, isothiazolyl, oxazolyl, isoxazolyl, triazolyl, and thiadiazolyl groups.

Preferably the group Het is linked to the methylene group shown in formula (1) by a carbon atom of the heterocyclic group which is adjacent to a nitrogen atom.

Examples of the group Het are 2- or 4-imidazolyl optionally substituted by $C_{1-4}$ alkyl (preferably methyl), halogen (preferably chlorine or bromine), trifluoromethyl or hydroxymethyl, 2-pyridyl optionally substituted by one or more (which can be the same or different) $C_{1-4}$ alkyl (preferably methyl), $C_{1-4}$ alkoxy (preferably methoxy), halogen (preferably chlorine or bromine), amino or hydroxy groups; 2-thiazolyl, 3-isothiazolyl optionally substituted by chlorine or bromine, 3-(1,2,5)-thiadiazolyl optionally substituted by chlorine or bromine, or 2-(5-amino-1,3,4-thiadiazolyl). Particular Het groups are 5-methyl-4-imidazolyl, 5-bromo-4-imidazolyl, 2-pyridyl, 3-methyl-2-pyridyl, 3-methoxy-2-pyridyl, 3-ethoxy-2-pyridyl, 3,4-dimethoxy-2-pyridyl, 3-fluoro-2-pyridyl, 3-chloro-2-pyridyl, 3-bromo-2-pyridyl, 3-iodo-2-pyridyl, 3-bromo-4-methyl-2-pyridyl and 2-thiazolyl.

Examples of heteroaryl groups B are pyridyl, furanyl, thienyl, thiazolyl, oxazolyl, isothiazolyl, imidazolyl,

pyrimidyl, pyrazinyl, pyridazyl, thiadiazolyl, quinolyl, isoquinolyl, 5,6,7,8-tetrahydroquinolyl, 1,3-dioxolopyridyl, benzimidazolyl and benzthiazolyl.

Particular heteroaryl groups for B are 2-furanyl, 2-thienyl, 2-pyridyl, 3-pyridyl, 4-pyridyl, 2-thiazolyl, 2-imidazolyl, 2-pyrimidyl, 2-pyrazyinl, 3-pyridazyl, 3-quinolyl and 1-isoquinolyl optionally substituted by one or more $C_{1-4}$ alkyl or $C_{1-4}$ alkoxy groups.

Specific heteroaryl groups for B are 2-furanyl, 3-pyridyl, 6-methyl-3-pyridyl, 5,6-dimethyl-3-pyridyl, 6-methoxy-3-pyridyl, 2-methoxy-4-pyridyl, 4-methoxy-2-pyridyl, 2-hydroxy-4-pyridyl 4-hydroxy-2-pyridyl and 6-hydroxy-3-pyridyl.

Specific substituted phenyl groups B are 3-chlorophenyl, 3,4-dichlorophenyl, 3-methoxyphenyl, 4-methoxyphenyl, 3,4-dimethoxyphenyl and 3,4,5-trimethoxyphenyl.

Specific meanings of A are methylene, 1,1-ethanediyl ($-CHCH_3-$), 1,2-ethanediyl ($-CH_2CH_2-$) and 1,3-propanediyl ($-CH_2CH_2CH_2-$).

Examples of groups displacable with thiol which L represents are hydroxy, acyloxy, $C_{1-4}$ alkoxy, chlorine, bromine and triarylphosphonium.

When L is acyloxy preferably it is acetoxy, methanesulphonyloxy or p-toluenesulphonyloxy.

When L is $C_{1-4}$ alkoxy preferably it is methoxy.

When L is triarylphosphonium preferably it is triphenylphosphonium.

Examples of phenolic hydroxy protecting groups are methoxymethyl, methylthiomethyl, tetrahydropyranyl, benzyl, acyl (particularly $C_{1-4}$ alkanoyl) and $C_{1-4}$ alkyl.

When L is hydroxy or $C_{1-4}$ alkoxy the reaction is carried out under acidic conditions for example in acetic acid or in aqueous hydrochloric or hydrobromic acid. When L is acyloxy, chlorine, bromine or triarylphosphonium, the reaction is carried out in the presence of a base for example an alkali metal $C_{1-4}$ alkoxide in particular sodium ethoxide.

Preferably L is hydroxy or methoxy except where Het or B is an acid-labile group when it is preferably chlorine or bromine.

Examples of acid addition salts of compounds of formula (1), (4) and (5) are those with hydrochloric and hydrobromic acids.

The reaction can be carried out in the presence of a solvent, the choice of which is not critical to the success of the reaction provided that it is substantially inert to the reagents and product. Where the reaction is carried out under acidic conditions in one of the acids given above by way of example, the solvent can be the acid itself.

Where the reaction is carried out under basic conditions using one of the bases given above by way of example, the solvent can be an excess of the corresponding $C_{1-4}$ alkanol.

The reaction can be carried out at moderate temperature for example up to the reflux temperature of the reaction mixture.

Preferably approximately equimolar amounts of the reagents are used, although an excess, for example a slight excess of from 1.1 to 1.5 molar equivalents or a larger excess of from 1.5 to 4 molar equivalents, of either reagent can be used. An excess of either reagent can be present at the start of the reaction or can be added during the course of the reaction.

The process of this invention is particularly useful for preparing compounds of formula (1) in which Het is a 2-thiazolyl, 5-methyl-4-imidazolyl, 5-bromo-4-imidazolyl, 3-bromo-2-pyridyl, 3-chloro-2-pyridyl, 3-methoxy-2-pyridyl or 3-hydroxy-2-pyridyl group, and in which n is 2.

Specifically the process is useful for preparing :-

(a) 2-[2-(5-methyl-4-imidazolylmethylthio)ethylamino]-5-(3-methoxybenzyl)-4-pyrimidone

(b) 2-[2-(5-methyl-4-imidazolylmethylthio)ethylamino]-5-(5-(1,3-benzodioxolyl)methyl)-4-pyrimidone

(c) 2-[2-(5-methyl-4-imidazolylmethylthio)ethylamino]-5-(3-pyridylmethyl)-4-pyrimidone and

(d) 2-[2-(5-methyl-4-imidazolylmethylthio)ethylamino]-5-(6-methyl-3-pyridylmethyl)-4-pyrimidone.

Compounds of formula (5) above can be prepared by reacting a compound of formula (6) :-

$$GS(CH_2)_nNH_2$$

(6)

where G is hydrogen or a thiol protecting group and n is as defined with reference to formula (1) with a compound

of formula (7) :-

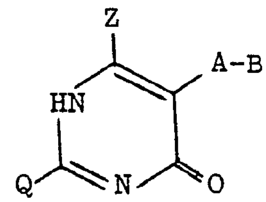

(7)

where Z, A and B are as defined with reference to formula (1) and Q is a group which is displacable with an amine and optionally converting the group G in the product when it is a thiol protecting group into hydrogen.

The products of this latter process have general formula (5a) :-

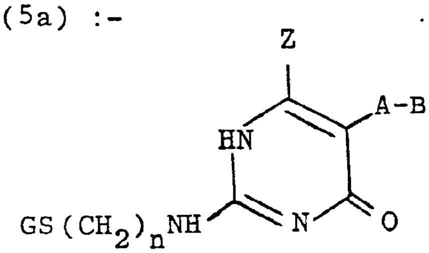

(5 a)

where A, B, Z and n are as defined with reference to formula (1) and G is as defined with reference to formula (6).

Specific compounds of formula (5a) are :-

2-(2-Mercaptoethylamino)-5-[5-(1,3-benzodioxolyl)methyl]-4-pyrimidone,

2-(2-Mercaptoethylamino)-5-(3-pyridylmethyl)-4-pyrimidone,

2-(2-Tritylthioethylamino)-5-[5-(1,3-benzodioxolyl)methyl]-4-pyrimidone,

2-(2-Mercaptoethylamino)-5-(6-methyl-3-pyridylmethyl)-4-pyrimidone and their acid addition salts.

Examples of groups displacable by amine which Q represents are nitroamino ($NO_2NH$-), $C_{1-4}$ alkylthio, benzylthio, chlorine and bromine.

Preferably Q is nitroamino or $C_{1-4}$ alkylthio particularly methylthio.

Examples of thiol protecting groups which G represents are trityl, 4-methoxybenzyl, ethylcarbamoyl and $-S(CH_2)NH_2$; in this latter case the thiol is protected as the disulphide.

The reaction can be carried out at an elevated temperature in the absence of a solvent, for example at 80° to 170°C, preferably 120° to 140°C, or in a solvent at an elevated temperature, for example at the reflux temperature of the reaction mixture. The choice of solvent is affected by solubility characteristics of the reagent.

Preferably the solvent is pyridine, a picoline or mixture of picolines, a $C_{1-4}$ alkanol, preferably ethanol or 1-propanol, an aqueous mixture of a $C_{1-4}$ alkanol, 1,2-ethanediol, a ketone, for example acetone or 2-butanone, or a polar aprotic solvent, for example dimethylformamide, dimethylacetamide, dimethylsulphoxide, hexamethylphosphoramide, sulpholane, acetonitrile or nitromethane. Particularly preferably the reaction is carried out in refluxing ethanol, refluxing 1-propanol or refluxing pyridine.

Where the thiol protecting group is acid labile (for example trityl) it is possible to carry out both the steps of removing the thiol-protecting group and the subsequent reaction with the compound of formula (4) simultaneously in the acid media referred to previously. That is to say the compound of formula (5) is generated in situ from a compound of formula (5b) :-

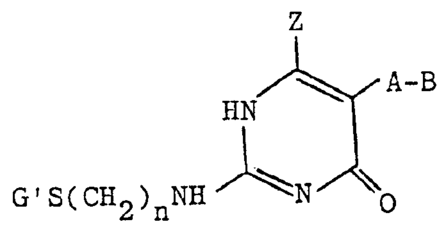

(5b)

where G' is an acid labile thiol protecting group and n, Z, A and B are as defined with reference to formula (1).

A particularly suitable acid medium for this purpose is acetic acid containing hydrogen chloride.

When the thiol-protecting group G is $-S(CH_2)_nNH_2$ the disulphide obtained can be converted into the corresponding compound of formula (5) by reduction for example with lithium aluminium hydride or by using a mercaptan for example dithiothreitol or using sodium sulphide.

The pyrimidones of formula (7) can be made by known methods for example where Q is nitroamino the compounds can be prepared by reacting nitroguanidine with a compound of formula (8) :-

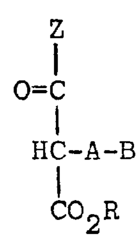

(8)

in which Z, A and B are as defined with reference to formula (1) and R is $C_{1-4}$ alkyl or aryl($C_{1-4}$ alkyl), in the presence of a base. Preferably this reaction is carried out in a $C_{1-4}$ alkanol with a sodium $C_{1-4}$ alkoxide as the base, and preferably at the boiling point of the reaction mixture.

The invention is illustrated by the following Examples.

Example 1

(i) A solution of ethyl 2-formyl-3-[5-(1,3-benzodioxolyl)]-propionate (7.5 g) in methanol (20 ml) was added to sodium methoxide in methanol (prepared from 0.689 g sodium and 50 ml methanol); nitroguanidine (3.12 g) was then added to the stirred mixture. The mixture was heated under reflux for 18 hours and evaporated to a residue which was dissolved in water (200 ml) and the solution was extracted with chloroform. The residual aqueous phase was adjusted to pH 5 with acetic acid and the white solid which precipitated was filtered off to give 2-nitroamino-5-[5-(1,3-benzodioxolyl)methyl)]-4-pyrimidone (4.08 g), m.p. 200-2°C. A sample recrystallised from aqueous acetic acid had m.p. 201.5-2.5°C.

(ii) Cysteamine (0.77 g) and 2-nitroamino-5-(5-(1,3-benzodioxolyl)methyl)-4-pyrimidone (2.90 g) were heated under reflux in pyridine (75 ml) for 16 hr in a nitrogen atmoxphere. The mixture was evaporated to dryness and concentrated hydrochloric acid (50 ml) was added to the residue which was warmed and stirred. The solid was filtered off and washed with water to give 2-(2-mercaptoethylamino)-5-[5-(1,3-benzodioxolyl) methyl]-4-pyrimidone hydrochloride (3.5 g). m.p. 148-152°C

(iii) A mixture of 2-(2-mercaptoethylamino)-5-[5-(1,3-benzodioxolyl)methyl]-4-pyrimidone hydrochloride (0.50 g), 4-hydroxymethyl-5-methylimidazole hydrochloride (0.22 g) and acetic acid (15 ml) was heated under

reflux for 16 hours, allowed to cool and filtered. The filtrate was evaporated to dryness and the residue recrystallised from ethanol to give 2-[2-(5-methyl-4-imidazolylmethylthio)-ethylamino]-5-[5-(1,3-benzodioxolyl)methyl]-4-pyrimidone dihydrochloride (0.2 g), m.p. 229-234$^{o}$C.

## Example 2

(i) A mixture of 6-methylpyridine-3-carboxaldehyde (51.57 g), malonic acid (44.30 g), piperidine (6 ml) and pyridine (300 ml) was stirred at 100$^{o}$C for 3 hours and was allowed to cool. The mixture was evaporated to dryness, water was added to the residue and the solid was filtered off and recrystallised from ethanol-acetic acid to give 3-(6-methyl-4-pyridyl)acrylic acid (41.25 g), m.p. 213.5-215.5$^{o}$C.

(ii) A stirred mixture of 3-(6-methyl-3-pyridyl)acrylic acid (50.70 g), dry ethanol (350 ml) and concentrated sulphuric acid (25 ml) was heated under reflux for 18 hours and ethanol ($\sim$ 250 ml) was removed by evaporation. The residue was poured into ice-aqueous ammonia and the mixture was extracted with ether. The ether extracts were washed with water and evaporated to an oil which crystallised on standing to give ethyl 3-(6-methyl-3-pyridyl)acrylate, m.p. 36-37$^{o}$C.

(iii) Ethyl 3-(6-methyl-3-pyridyl)acrylate (60.36 g) was hydrogenated in ethanol at 35$^{o}$ and 355 kPa using palladium-on charcoal catalyst (10%, 1.0 g). The mixture was filtered and the filtrate was evaporated to give ethyl 3-(6-methyl-3-pyridyl)propionate as an oil.

(iv) A mixture of ethyl 3-(6-methyl-3-pyridyl)-propionate (1.31 g) and ethyl formate (7.43 g) was added dropwise to a stirred suspension of sodium hydride (50% dispersion in oil, 4.07 g) in dry 1,2-dimethoxyethane (24 ml) maintained at 0°C. The mixture was allowed to warm to room temperature, stirred overnight and poured into ice-water (300 g). The mixture was extracted with ether, the aqueous phase was adjusted to pH 5.4 with hydrochloric acid, and the solid which separated was collected to give ethyl 2-formyl-3-(6-methyl-3-pyridyl)propionate (10.5 g, 70%), m.p. 142-4°C.

(v) A solution of ethyl 2-formyl-3-(6-methyl-3-pyridyl)-propionate (1.55 g) in methanol (20 ml) was added to a stirred solution of sodium methoxide (from 0.161 g sodium) in methanol (20 ml). Dried nitroguanidine (0.73 g) was added and the mixture was heated under reflux overnight and evaporated to dryness. The residue was dissolved in water (50 ml) and the solution was extracted with chloroform and the aqueous phase was adjusted to pH 5 with acetic acid. The solid which precipitated was filtered off and recrystallised from methanol-acetic acid to give 2-nitroamino-5-(6-methyl-3-pyridylmethyl)-4-pyrimidone (0.5 g, 27%), m.p. 215-6°C (decomp).

(vi) A mixture of cysteamine (1.54 g), 2-nitroamino-5-(6-methyl-3-pyridylmethyl)-4-pyrimidone (5.22 g) and dry pyridine (150 ml) was heated under an atmosphere of nitrogen at 100°C for 16 hours and then heated under reflux for 6 hours. This cooled mixture was filtered to give 2-(2-mercaptoethylamino)-5-(6-methyl-3-pyridylmethyl)-4-pyrimidone (2.6 g) m.p. 180-185°C.

(vii) 2-(2-Mercaptoethylamino)-5-(6-methyl-3-pyridylmethyl)-4-pyrimidone and 4-hydroxymethyl-5-methylimidazole are heated under reflux in aqueous hydrobromic acid to give 2-[2-(5-methyl-4-imidazolylmethylthio)-ethylamino]-5-(6-methyl-3-pyridylmethyl)-4-pyrimidone trihydrobromide.

## Example 3

(a) 2-Tritylthioethylamine (3.19 g) and 2-nitroamino-5-(5-(1,3-benzodioxolyl)methyl)-4-pyrimidone (2.90 g) are heated under reflux in ethanol to give 2-(2-tritylthioethylamino)-5-[5-(1,3-benzodioxolyl)methyl]-4-pyrimidone (2.95 g).

(b) 2-(2-Tritylthioethylamino)-5-(5-(1,3-benzodioxolyl)-methyl)-4-pyrimidone (0.41 g) and 4-hydroxymethyl-5-methylimidazole hydrochloride (0.11 g) are heated under reflux in acetic acid containing hydrogen chloride to give 2-[2-(5-methyl-4-imidazolylmethylthio)ethylamino]-5-[5-(1,3-benzodioxolyl)methyl]-4-pyrimidone (0.07 g of dihydrochloride).

## Example 4

Cystamine is reacted with 2-nitroamino-5-(6-methyl-3-pyridylmethyl)-4-pyrimidone by heating in ethanol to give 2-[5-(3-pyridylmethyl)-4-oxo-2-pyrimidylamino]ethyl disulphide which is reduced with dithiothreitol to give 2-(2-mercaptoethylamino)-5-(6-methyl-3-pyridylmethyl)-4-pyrimidone which is heated under reflux with 3-bromo-2-hydroxymethylpyridine hydrobromide in acetic acid to give 2-[2-(3-bromo-2-pyridylmethylthio)ethylamino]-5-(6-methyl-3-pyridylmethyl)-4-pyrimidone.

WHAT WE CLAIM IS :-

1. A process for preparing a compound of formula (1) :-

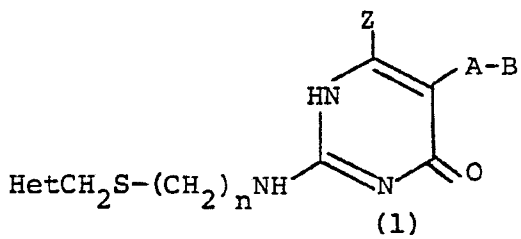

(1)

and acid addition salts thereof where Het is an optionally substituted 5- or 6- membered fully unsaturated nitrogen containing heterocyclic group in which the substituents (which can be the same or different) are one or more $C_{1-4}$ alkyl, trifluoromethyl, halogen, hydroxy, $C_{1-4}$ alkoxy or amino groups; n is 2 or 3; Z is hydrogen or $C_{1-4}$ alkyl; A is $C_{1-5}$ alkylene optionally interrupted with oxygen or sulphur and B is hydrogen, methyl, $C_{3-6}$ cycloalkyl, an optionally substituted heteroaryl group where the substituents (which can be the same or different) are one or more $C_{1-4}$ alkyl, $C_{1-4}$ alkoxy or hydroxy groups; or B is a naphthyl, 5-or 6-(2,3-dihydro-1, 4-benzodioxinyl) or a 4- or 5-(1,3-benzodioxolyl) group or an optionally substituted phenyl group where the substituents (which can be the same or different) are one or more $C_{1-4}$ alkyl, $C_{1-4}$ alkoxy, halogen, aryl-($C_{1-4}$ alkoxy), hydroxy, $C_{1-4}$ alkoxy-$C_{1-4}$ alkoxy, trifluoromethyl, di($C_{1-4}$ alkyl) amino, phenoxy, halophenyl, or $C_{1-4}$ alkoxyphenyl groups <u>characterised in that</u> a compound of formula (4) :-

$$Het\text{-}CH_2L$$

(4)

or an acid addition salt thereof where Het is as defined with reference to formula (1) provided that any substituents which interfere with the reaction are protected and L is a group displacable with thiol is reacted with a compound of formula (5) :-

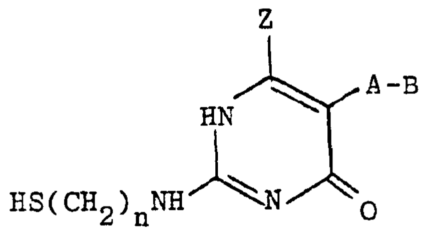

(5)

or an acid addition salt thereof where n, Z, A and B are as defined with reference to formula (1) provided that any phenolic hydroxy groups within B are optionally protected, thereafter removing any protecting groups and optionally converting the product into an addition salt.

2. A process as claimed in claim 1 characterised in that L is hydroxy, acetoxy, methanesulphonyloxy, p-toluenesulphonyloxy, methoxy, chlorine, bromine, or triphenylphosphonium.

3. A process as claimed in claim 1 or claim 2 characterised in that L is hydroxy or $C_{1-4}$ alkoxy and the reaction is carried out under acidic conditions.

4. A process as claimed in claim 1 or claim 2 characterised in that Het or B is acid labile, L is chlorine or bromine and the reaction is carried out in the presence of base.

5. A process as claimed in claim 1 or claim 2 characterised in that the product is 2-[2-(5-methyl-4-imidazolylmethylthio)ethylamino]-5-(3-methoxybenzyl)-4-pyrimidone, 2-[2-(5-methyl-4-imidazolylmethylthio)ethylamino]-5-[5-(1,3-benzodioxolyl)methyl]-4-pyrimidone and 2-[2-(5-methyl-4-imidazolylmethylthio)ethylamino]-5-(3-pyridylmethyl)-4-pyrimidone and 2-[2-(5-methyl-4-imidazolylmethylthio)ethylamino]-5-(6-methyl-3-pyridylmethyl)-4-pyrimidone.

6. A process as claimed in any one of claims 1 to 3 and 5 characterised in that the reaction is carried out in an acidic medium and the compound of formula (5) is generated *in situ* from a compound of formula (5b) :-

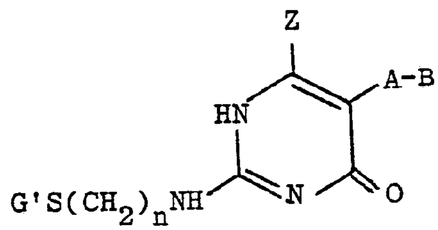

(5b)

where n, Z, A and B are as defined with reference to formula (1) and G' is an acid labile thiol-protecting group.

0024873

European Patent Office

# EUROPEAN SEARCH REPORT

Application number

EP 80 30 2864

## DOCUMENTS CONSIDERED TO BE RELEVANT

| Category | Citation of document with indication, where appropriate, of relevant passages | Relevant to claim |
|---|---|---|
| | METHODEN DER ORGANISCHEN CHEMIE , Houben-Weyl, Band IX, Schwefel-, Selen-, Tellur-Verbindungen, 1955, pages 103-117 Stuttgart, DE. * Pages 103,107,114-117 * -- | 1-4 |
| D,P | EP - A - 0 004 793 (SMITH-KLINE) * Page 1; page 16, iii * -- | 1,4 |
| P | EP - A - 0 013 071 (SMITH KLINE) ---- | 1-6 |

CLASSIFICATION OF THE APPLICATION (Int. Cl.[3])

C 07 D 401/12
401/14
403/12
403/14
405/12
405/14

TECHNICAL FIELDS SEARCHED (Int. Cl.[3])

C 07 D 401/00
403/00
405/00
239/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 03-12-1980 | FRANCOIS |

EPO Form 1503.1 06.78